(19) Europäisches Patentamt
European Patent Office
Office européen des brevets

(11) Veröffentlichungsnummer : **0 306 521 B1**

(12) # EUROPÄISCHE PATENTSCHRIFT

(45) Veröffentlichungstag der Patentschrift :
**16.10.91 Patentblatt 91/42**

(51) Int. Cl.$^5$ : **C07C 43/225,** C07D 239/26, C07D 213/30, C07D 213/65, C09K 19/30, C09K 19/34

(21) Anmeldenummer : **88903217.3**

(22) Anmeldetag : **25.03.88**

(86) Internationale Anmeldenummer :
**PCT/EP88/00246**

(87) Internationale Veröffentlichungsnummer :
**WO 88/07516 06.10.88 Gazette 88/22**

(54) **ETHINDERIVATE ALS KOMPONENTE FLÜSSIGKRISTALLINER PHASEN.**

(30) Priorität : **27.03.87 DE 3710069**

(43) Veröffentlichungstag der Anmeldung :
**15.03.89 Patentblatt 89/11**

(45) Bekanntmachung des Hinweises auf die Patenterteilung :
**16.10.91 Patentblatt 91/42**

(84) Benannte Vertragsstaaten :
**CH DE FR GB LI**

(56) Entgegenhaltungen :
**EP-A- 0 111 695**
**GB-A- 2 155 465**

(73) Patentinhaber : **MERCK PATENT GESELLSCHAFT MIT BESCHRÄNKTER HAFTUNG**
**Frankfurter Strasse 250 Postfach 4119**
**W-6100 Darmstadt (DE)**

(72) Erfinder : **KURMEIER, Hans-Adolf**
**Hinter der Schule 3a**
**W-6109 Mühltal 1 (DE)**
Erfinder : **REIFFENRATH, Volker**
**Jahnstr. 18**
**W-6101 Ro dorf (DE)**
Erfinder : **POETSCH, Eike**
**Am Buchwald 4**
**W-6109 Mühltal (DE)**
Erfinder : **KRAUSE, Joachim**
**Samuel-Morse-Str. 14**
**W-6110 Dieburg (DE)**
Erfinder : **WEBER, Georg**
**Wilhelm-Leuschner-Str. 38**
**W-6106 Erzhausen (DE)**

**EP 0 306 521 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft Ethinderivate der Formel I

$$R^1\text{-}(A^1\text{-}Z^1)_m\text{-}A^3\text{-}C\equiv C\text{-}A^4\text{-}(Z^2\text{-}A^2)_n\text{-}R^2 \quad I$$

worin

$R^1$ einen unsubstituierten, einen einfach durch -CN oder einen mindestens einfach durch Halogen substituierten Alkyl- oder Alkenylrest mit 1 bis 15 C-Atomen, wobei in diesen Resten auch eine oder mehrere $CH_2$-Gruppen jeweils unabhängig voneinander durch -O-, -S-, -CO-, -O-CO-, -O-COO-, -CO-O- oder -C≡C- so ersetzt sein können, daß Heteroatome nicht direkt miteinander verknüpft sind, H, Halogen, -CN oder -NCS,

$R^2$ $-OCF_3$, $-OC_2F_5$ oder $-OC_2F_4H$,

$A^1$ und $A^2$ jeweils unabhängig voneinander einen

a) 1,4-Phenylenrest, worin auch eine oder mehrere CH-Gruppen durch N ersetzt sein können,

b) trans-1,4-Cyclohexylenrest, worin auch eine oder zwei nicht benachbarte $CH_2$-Gruppen durch -O- und/oder -S-ersetzt sein können,

c) Rest aus der Gruppe 1,4-Cyclohexenylen, 1,4-Cyclohexadienylen oder 1,4-Bicyclo-(2.2.2)-octylen, wobei die Reste a) und b) ein- oder mehrfach durch Halogen, Cyano und/oder $CH_3$ substituiert sein können,

$Z^1$ und $Z^2$ jeweils unabhängig voneinander -CO-O-, -O-CO-, $-CH_2O-$, $-OCH_2-$, $-CH_2CH_2-$, -C≡C- oder eine Einfachbindung,

m und n jeweils unabhängig voneinander 0 oder 1,

und

$A^3$ und $A^4$ jeweils unabhängig voneinander einen

a) 1,4-Phenylen- oder 4,4'-Biphenylenrest, worin auch eine oder mehrere CH-Gruppen durch N ersetzt sein können,

b) trans-1,4-Cyclohexylenrest,

c) Rest aus der Gruppe 1,4-Cyclohexenylen, 1,4-Cyclohexadienylen oder 1,4-Bicyclo(2.2.2)octylen, wobei die Reste a) und b) ein- oder mehrfach durch Halogen, Cyano und/oder $CH_3$ substituiert sein können,

bedeutet, mit der Maßgabe daß, in mindestens einer der Gruppen $A^3$ oder $A^4$ mindestens eine CH-Gruppe durch N ersetzt ist und/oder mindestens eine der Gruppen $A^3$ und $A^4$ trans-1,4-Cyclohexylen oder 1,4-Bicyclo(2.2.2)octylen ist und/oder mindestens eine der Gruppen $A^1$, $A^2$, $A^3$ und $A^4$ 1,4-Cyclohexenylen oder 1,4-Cyclohexadienylen ist.

Der Einfachheit halber bedeuten im folgenden Phe eine unsubstituierte 1,4-Phenylengruppe, PheX eine substituierte 1,4-Phenylengruppe (wobei X Halogen, CN und/oder $CH_3$ bedeutet), Cyc eine 1,4-Cyclohexylengruppe, Che eine 1,4-Cyclohexenylengruppe, Cha eine 1,4-Cyclohexadienylengruppe, Dio eine 1,3-Dioxan-2,5-diylgruppe, Dit eine 1,3-Dithian-2,5-diylgruppe, Pyd eine Pyridin-2,5-diylgruppe, Pyr eine Pyrimidin-2,5-diylgruppe, Pyz eine Pyrazin-2,5-diylgruppe, Pyn eine Pyridazin-3,6-diylgruppe, Bco eine 1,4-Bicyclo(2.2.2)octylengruppe und Biphe eine 4,4'-Biphenylylgruppe, worin auch eine oder mehrere CH-Gruppen durch N ersetzt sein können.

Die Verbindungen der Formel I können als Komponenten flüssigkristalliner Phasen verwendet werden, insbesondere für Displays, die auf dem Prinzip der verdrillten Zelle, dem Guest-Host-Effekt, dem Effekt der Deformation aufgerichteter Phasen oder dem Effekt der dyamischen Streuung beruhen.

Ähnliche Verbindungen sind z.B. aus der EP 111695-A für Displays, die nach dem Zwei-Frequenz-Verfahren arbeiten, bekannt.

Der Erfindung lag die Aufgabe zugrunde, neue stabile flüssigkristalline oder mesogene Verbindungen aufzufinden, die als Komponenten flüssigkristalliner Phasen geeignet sind. Diese Aufgabe wurde durch die Bereitstellung der Verbindungen der Formel I gelöst.

Es wurde gefunden, daß die Verbindungen der Formel I als Komponenten flüssigkristalliner Phasen vorzüglich geeignet sind. Insbesondere zeichnen sich diese Verbindungen durch eine positive dielektrische Anisotropie und eine hohe Doppelbrechung aus.

Die Verbindungen der Formel I sind somit vorzugsweise für die Verwendung als Komponenten flüssigkristalliner Phasen für SBE- oder OMI-Anwendungen geeignet.

Überraschend zeigte sich, daß der Zusatz von Verbindungen der Formel I flüssigkristalline Phasen mit breiten, nematischen Bereichen liefert.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüs-

sigkristallinen Basismaterialien aus anderen Verbindungsklassen zugesetzt werden, um beispielsweise die dielektrische und/oder optische Anisotropie eines solchen Dielektrikums zu optimieren.

Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der formel I sowie die Verwendung dieser Verbindungen als Komponenten flüssigkristalliner Phasen. Weiterhin sind Gegenstand der Erfindung flüssigkristalline Phasen mit einem Gehalt an mindestens einer Verbindung der Formel I sowie Flüssigkristall-Anzeigeelemente, die derartige Phasen enthalten.

Vor- und nachstehend haben $R^1$, $A^1$, $Z^1$, m, $A^3$, $A^4$, $Z^2$, $A^2$, n und $R^2$ die angegebene Bedeutung, sofern nicht ausdrücklich etwas anderes vermerkt ist.

Die Verbindungen der Formel I umfassen dementsprechend . Verbindungen der Teilformeln Ia (mit zwei Ringen), Ib bis Ie (mit drei Ringen) und If bis Ii (mit vier Ringen):

$R^1$-$A^3$-C≡C-$A^4$-$R^2$ Ia

$R^1$-$A^3$-C≡C-$A^4$-$A^2$-$R^2$ Ib

$R^1$-$A^3$-C≡C-$A^4$-$Z^2$-$A^2$-$R^2$ Ic

$R^1$-$A^1$-$A^3$-C≡C-$A^4$-$R^2$ Id

$R^1$-$A^1$-$Z^1$-$A^3$-C≡C-$A^4$-$R^2$ Ie

$R^1$-$A^1$-$Z^1$-$A^3$-C≡C-$A^4$-$Z^2$-$A^2$-$R^2$ If

$R^1$-$A^1$-$A^3$-C≡C-$A^4$-$Z^2$-$A^2$-$R^2$ Ig

$R^1$-$A^1$-$Z^1$-$A^3$-C≡C-$A^4$-$A^2$-$R^2$ Ih

$R^1$-$A^1$-$A^3$-C≡C-$A^4$-$A^2$-$R^2$ Ii

Die bevorzugten Verbindungen der Teilformel Ia umfassen solche der Teilformeln Iaa bis Ian:

$R^1$-Cyc-C≡C-Phe-$R^2$ Iaa

$R^1$-Cyc-C≡C-PheX-$R^2$ Iab

$R^1$-Che-C≡C-Phe-$R^2$ Iac

$R^1$-Cha-C≡C-Phe-$R^2$ Iad

$R^1$-Pyd-C≡C-Phe-$R^2$ Iae

$R^1$-Pyr-C≡C-Phe-$R^2$ Iaf

$R^1$-Pyz-C≡C-Phe-$R^2$ Iag

$R^1$-Pyn-C≡C-Phe-$R^2$ Iah

$R^1$-Bco-C≡C-Phe-$R^2$ Iai

$R^1$-Cyc-C≡C-Biphe-$R^2$ Iak

$R^1$-Pyd-C≡C-Biphe-$R^2$ Ial

$R^1$-Che-C≡C-PheX-$R^2$ Iam

$R^1$-Cha-C≡C-PheX-$R^2$ Ian

Darunter sind diejenigen der Formeln Iaa, Iab, Iac, Iae, Iaf, Iak und Iam besonders bevorzugt.

Die bevorzugten Verbindungen der Teilformeln Ib, Ic, Id und Ie umfassen solche der Teilformeln I1 bis I22:

$R^1$-Cyc-C≡C-$A^4$-$Z^2$-$A^2$-$R^2$ I1

$R^1$-Che-C≡C-$A^4$-$Z^2$-$A^2$-$R^2$ I2

$R^1$-Cha-C≡C-$A^4$-$Z^2$-$A^2$-$R^2$ I3

$R^1$-Bco-C≡C-$A^4$-$Z^2$-$A^2$-$R^2$ I4

$R^1$-Pyd-C≡C-$A^4$-$Z^2$-$A^2$-$R^2$ I5

$R^1$-Pyr-C≡C-$A^4$-$Z^2$-$A^2$-$R^2$ I6

$R^1$-Pyz-C≡C-$A^4$-$Z^2$-$A^2$-$R^2$ I7

$R^1$-Pyn-C≡C-$A^4$-$Z^2$-$A^2$-$R^2$ I8

$R^1$-$A^3$-C≡C-Cyc-$Z^2$-$A^2$-$R^2$ I9

$R^1$-$A^3$-C≡C-Che-$Z^2$-$A^2$-$R^2$ I10

$R^1$-$A^3$-C≡C-Cha-$Z^2$-$A^2$-$R^2$ I11

$R^1$-$A^3$-C≡C-Bco-$Z^2$-$A^2$-$R^2$ I12

$R^1$-Biphe-C≡C-$A^4$-$Z^2$-$A^2$-$R^2$ I13

$R^1$-$A^3$-C≡C-Biphe-$Z^2$-$A^2$-$R^2$ I14

$R^1$-$A^3$-C≡C-Pyd-$Z^2$-$A^2$-$R^2$ I15

$R^1$-$A^3$-C≡C-Pyr-$Z^2$-$A^2$-$R^2$ I16

$R^1$-$A^3$-C≡C-PyZ-$Z^2$-$A^2$-$R^2$ I17

$R^1$-$A^3$-C≡C-Pyn-$Z^2$-$A^2$-$R^2$ I18

$R^1$-$A^3$-C≡C-$A^4$-$Z^2$-Che-$R^2$ I19
$R^1$-$A^3$-C≡C-$A^4$-$Z^2$-Cha-$R^2$ I20
$R^1$-Che-$Z^1$-$A^3$-C≡C-$A^4$-$R^2$ I21
$R^1$-Cha-$Z^1$-$A^3$-C≡C-$A^4$-$R^2$ I22

Darunter sind diejenigen der Formeln I1, I2, I5, I6, I9, I10, I13, I14, I15, I18, I19 und I20 besonders bevorzugt.

Die bevorzugten Verbindungen der Teilformeln If, Ig, Ih und Ii umfassen solcher der Teilformeln I23 bis I33:

$R^1$-$A^1$-$Z^1$-Cyc-C≡C-$A^4$-$Z^2$-$A^2$-$R^2$ I23
$R^1$-$A^1$-$Z^1$-Che-C≡C-$A^4$-$Z^2$-$A^2$-$R^2$ I24
$R^1$-$A^1$-$Z^1$-Cha-C≡C-$A^4$-$Z^2$-$A^2$-$R^2$ I25
$R^1$-$A^1$-$Z^1$-Bco-C≡C-$A^4$-$Z^2$-$A^2$-$R^2$ I26
$R^1$-$A^1$-$Z^1$-Pyd-C≡C-$A^4$-$Z^2$-$A^2$-$R^2$ I27
$R^1$-$A^1$-$Z^1$-Pyr-C≡C-$A^4$-$Z^2$-$A^2$-$R^2$ I28
$R^1$-$A^1$-$Z^1$-Pyz-C≡C-$A^4$-$Z^2$-$A^2$-$R^2$ I29
$R^1$-$A^1$-$Z^1$-Pyn-C≡C-$A^4$-$Z^2$-$A^2$-$R^2$ I30
$R^1$-$A^1$-$Z^1$-Biphe-C≡C-$A^4$-$Z^2$-$A^2$-$R^2$ I31
$R^1$-Che-$Z^1$-$A^3$-C≡C-$A^4$-$Z^2$-$A^2$-$R^2$ I32
$R^1$-Cha-$Z^1$-$A^3$-C≡C-$A^4$-$Z^2$-$A^2$-$R^2$ I33

In den vor- und nachstehenden Formeln bedeutet $R^1$ vorzugsweise Alkyl, Alkoxy oder eine andere Oxaalkylgruppe.

Ferner bevorzugt für $R^1$ sind Alkenylgruppen oder einfach durch -CN substituierte Alkylgruppen. Durch Halogen substituierte Alkylgruppen sind ebenfalls bevorzugt.

Halogen bedeutet Fluor, Chlor oder Brom. Bevorzugt ist eine Substitution durch Fluor oder Chlor.

Ferner hat $R^1$ auch die Bedeutung Halogen, -CN oder -NCS.

$A^1$ und $A^2$ sind bevorzugt Cyc, PheX oder Phe, ferner auch Che oder Cha. PheX bedeutet vorzugsweise Monosubstitution durch F, Cl oder CN.

Weiterhin bevorzugt stellen $A^1$ und $A^2$ eine Bco-, Pyd-, Dio- oder Pyr-Gruppe dar.

$A^3$ und $A^4$ bedeuten bevorzugt Cyc, Pyd, Pyr, Che, Phe oder Cha, ferner bevorzugt Bco, PheX, Biphe, Pyn oder Pyz.

In mindestens einer der Gruppen $A^3$ oder $A^4$ ist mindestens eine CH-Gruppe durch N ersetzt und/oder mindestens eine der Gruppen $A^3$ und $A^4$ bedeutet Cyc oder Bco und/oder mindestens eine der Gruppen $A^1$, $A^2$, $A^3$ oder $A^4$ ist Che oder Cha. Vorzugsweise bedeutet nur eine der Gruppen $A^3$ oder $A^4$ einen Pyridin-, Cyclohexyl- oder Pyrimidinrest.

m und n bedeuten jeweils unabhängig voneinander 0 oder 1. Vorzugsweise ist m + n = 1.

$Z^1$ und $Z^2$ bedeuten bevorzugt Einfachbindungen, -CO-O- oder -O-CO-. In zweiter Linie bevorzugt sind -$CH_2CH_2$-, -C≡C-, -$OCH_2$- oder -$CH_2O$-.

Falls $R^1$ einen Alkylrest bedeutet, in dem auch eine ("Alkoxy" bzw. "Oxaalkyl") oder zwei ("Alkoxyalkoxy" bzw. "Dioxaalkyl") nicht benachbarte $CH_2$-Gruppen durch O-Atome ersetzt sein können, so kann er geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy, Tetradecoxy, Pentadecoxy, 2-, 3-, 4-, 5-, 6- oder 7- Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Besonders bevorzugt sind auch Flügelgruppen in denen eine $CH_2$-Gruppe durch eine -C≡C-Gruppe ersetzt ist.

Verbindungen der Formel I mit verzweigter Flügelgruppe $R^1$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind.

Verzweigte Gruppen dieser Art enthalten in der Regel nicht mehr als eine Kettenverzweigung. Bevorzugte verzweigte Rest sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy (= 2-Octyloxy), 2-Oxa-3-methylbutyl, 3-Oxa-4-methylpentyl, 4-Methylhexyl, 2-Nonyl, 2-Decyl, 2-Dodecyl, 6-Methyloctoxy, 6-Methyloctanoyloxy, 5-Methylheptyloxycarbonyl, 2-Methylbutyryloxy, 3-Methylvaleryloxy, 4-Methylhexanoyloxy, 2-Chlorpropionyloxy, 2-Chlor-3-methylbutyryloxy, 2-Chlor-4-methylvaleryloxy, 2-Chlor-3-methylvaleryloxy, 2-Methyl-3-oxapentyl, 2-Methyl-3-oxahexyl.

Bei Verbindungen mit verzweigten Flügelgruppen umfaßt Formel I sowohl die optischen Antipoden als auch Racemate sowie deren Gemische.

Unter den Verbindungen der Formel I und deren Unterformeln sind diejenigen bevorzugt, in denen mindestens einer der darin enthaltenen Reste eine der angegebenen bevorzugten Bedeutungen hat.

Die 1,4-Cyclohexenylen-Gruppe hat vorzugsweise folgende Strukturen:

Die 1,4-Cyclohexadienylen-Gruppe weist bevorzugt folgende Struktur auf:

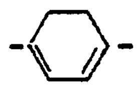

Besonders bevorzugte kleinere Gruppen von Verbindungen sind diejenigen der Formeln 1 bis 43:

Alkyl-Cyc-C≡C-Phe-$OCF_3$ 1
Alkyl-Cyc-C≡C-PheX-$OCF_3$ 2
Alkyl-Che-C≡C-Phe-$OCF_3$ 3
Alkyl-Cha-C≡C-Phe-$OCF_3$ 4
$CF_3O$-Phe-C≡C-Pyd-Alkyl 5
Alkyl-Cyc-C≡C-Phe-$OC_2F_4H$ 6
Alkyl-Cyc-C≡C-Phe-$OC_2F_5$ 7
Alkyl-Pyd-C≡C-Phe-$OC_2F_4H$ 8
Alkyl-Pyd-C≡C-Phe-$OC_2F_5$ 9
Alkyl-Cyc-Cyc-C≡C-Phe-$OC_2F_4H$ 10
Alkyl-Cyc-Cyc-C≡C-Phe-$OC_2F_5$ 11
Alkyl-Phe-Cyc-C≡C-Phe-$OC_2F_4H$ 12
Alkyl-Phe-Cyc-C≡C-Phe-$OC_2F_5$ 13
Alkyl-Phe-Pyd-C≡C-Phe-$OC_2F_4H$ 14
Alkyl-Phe-Pyd-C≡C-Phe-$OC_2F_5$ 15
Alkyl-Cyc-C≡C-Biphe-$OCF_3$ 16
Alkoxy-Pyd-C≡C-Phe-$OCF_3$ 17
Alkyl-Bco-C≡C-Phe-$OCF_3$ 18
$CH_3O$-Phe-C≡C-Pyn-Alkyl 19
Alkyl-Cyc-C≡C-Phe-COO-Phe-$OCF_3$ 20
Alkyl-Che-C≡C-Phe-OCO-Phe-$OCF_3$ 21
$R^1$-Cha-Phe-C≡C-Phe-$OCF_3$ 22
$R^1$-Cyc-$CH_2CH_2$-Cyc-C≡C-Phe-$OCF_3$ 23
$R^1$-Phe-Pyd-C≡C-Phe-$OCF_3$ 24
$R^1$-Che-Phe-C≡C-Phe-$OCF_3$ 25
$R^1$-Pyr-C≡C-Phe-OCO-Phe-$OCF_3$ 26
$R^1$-Phe-Phe-C≡C-Cyc-Phe-$OCF_3$ 27
$R^1$-Phe-COO-Phe-C≡C-Pyd-Phe-$OCF_3$ 28
$R^1$-Phe-$OCH_2$-Phe-C≡C-Cyc-Phe-$OCF_3$ 29
$R^1$-Cyc-Cyc-C≡C-Pyn-Phe-$OCF_3$ 30
Alkyl-Che-Phe-C≡C-Phe-$OCF_3$ 31
$R^1$-Che-Phe-C≡C-Phe-Phe-$OCF_3$ 32
$R^1$-Phe-Bco-C≡C-Phe-COO-Phe-$OCF_3$ 33
$R^1$-Cyc-Cha-C≡C-Phe-Phe-$OCF_3$ 34
$R^1$-Cyc-Cyc-C≡C-Pyr-Phe-$OCF_3$ 35
$R^1$-Phe-Phe-C≡C-Pyz-Phe-$OCF_3$ 36
$R^1$-Phe-Che-C≡C-Phe-Phe-$OCF_3$ 37

Alkyl-Cyc-C≡C-Cyc-C≡C-Phe-$OCF_3$ 38
Alkyl-Cyc-,Cyc-C≡C-Phe-$OCF_3$ 39
Alkyl-Phe-Cyc-C≡C-Phe-$OCF_3$ 40
Alkyl-Pyr-C≡C-Phe-$OCF_3$ 41
Alkyl-Pyr-C≡C-Phe-$OC_2F_4H$ 42
Alkyl-Pyr-C≡C-Phe-$OC_2F_5$ 43

Bevorzugt sind auch Verbindungen der Formel 1, in denen eine der Gruppen $A^1$, $A^2$, $A^3$ und $A^4$ eine 2,3-Dihalogen-1,4-phenylengruppe darstellt. Halogen bedeutet darin Fluor, Chlor oder Brom. Bevorzugter Substituent ist Fluor.

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den verbindungen der Formel I umsetzt.

So können die verbindungen der Formel I mit einer Dreifachbindung hergestellt werden, indem man die entsprechenden Stilbene bromiert und anschließend einer Dehydrohalogenierung unterwirft. Dabei kann man an sich bekannte, hier nicht näher erwähnte Varianten dieser Umsetzung anwenden.

Die Stilbene können hergestellt werden durch Umsetzung eines 4-substituierten Benzaldehyds mit einem entsprechenden Phosphorylid nach Wittig oder durch Umsetzung von einem 4-substituierten Phenylethylen mit einem entsprechenden Brombenzolderivat nach Heck.

Eine weitere Möglichkeit zur Herstellung der C-C-Dreifachbindung besteht darin, eine Verbindung, die sonst der Formel I entspricht, aber an Stelle der -C≡C-Bindung eine $-CH_2-CO-$Gruppe enthält, entweder mit einem anorganischen Säurechlorid umzusetzen, und die dann entstandene Gruppe $-CH_2-CCl_2-$ in Gegenwart einer Base zu dehydrohalogenieren, oder mit Semicarbazid und Selendioxid umzusetzen. Anschließend wird in Gegenwart von Methyllithium unter Erwärmen die Dreifachbindung eingeführt.

Ferner besteht die Möglichkeit, ein entsprechendes Benzilderivat mit Hydrazin und anschließend mit HgO in das Ethinderivat umzuwandeln.

Verbindungen der Formel I können auch hergestellt werden über die Kopplung von Alkinyl-Zink-Verbindungen mit Arylhalogeniden analog dem von A.O. King, E. Negishi, F.J. Villani und A. Silveira in J.Org.Chem. 43 (1978) 358 beschriebenen Verfahren.

Verbindungen der Formel I können auch über die Fritsch-Buttenberg-Wiechell-Umlagerung (Ann. 279, 319, 327, 332, 1894) hergestellt werden, bei der 1,1-Diaryl-2-halogenethylene umgelagert werden zu Diarylacetylenen in Gegenwart starker Basen.

Verbindungen der Formel I können weiterhin hergestellt werden aus 4-substituierten Phenyl- oder Cyclohexylacetylenen und Arylhalogeniden in Gegenwart eines Palladiumkatalysators, z.B. Bis(triphenylphosphin)-palladium(II)chlorid, und Kupfer(I)-jodid (beschrieben in Synthesis (1980) 627 oder Tetrahedron Letters 27 (1986) 1171).

Vorzugsweise werden als Ausgangsverbindungen dabei die entsprechenden Trifluormethoxybenzolderivate eingesetzt. Die Trifluormethoxygruppe kann beispielsweise eingeführt werden durch Umsetzung eines entsprechenden Phenolderivats mit wasserfreier Flußsäure in einem Autoklaven bei 0° in Tetrachlormethan, oder man geht von dem bekannten 4-Trifluormethoxybenzaldehyd oder 4-Tetrafluorethoxybenzaldehyd aus.

Verbindungen der Formel I sind weiterhin erhältlich, indem man an ein entsprechendes Cyclohexenderivat eine Verbindung der Formel HX (Fluor-, Chlor-, Brom- oder Cyanwasserstoff) anlagert.

Diese Anlagerung gelingt z.B. in Gegenwart eines inerten Lösungsmittels, z.B. eines halogenierten Kohlenwasserstoffs wie $CH_2Cl_2$ oder $CHCl_3$, eines Nitrils wie Acetonitril oder eines Amids wie Dimethylformamid (DMF) bei Temperaturen zwischen etwa -10 und +150° und Drucken zwischen etwa 1 und 100 bar. Ein Zusatz von Katalysatoren kann günstig sein, z.B. kann eine HCN-Anlagerung durch Zusatz von Palladium-bis-[2,3-O-isopropyliden-2,3-dihydroxy-1,4-bis-(diphenylphosphino)-butan] katalysiert werden.

Ester der Formel I (-CO-O- oder -O-CO-Gruppe in $R^1$ und/oder $Z^2$ und/oder $Z^1$ = -CO-O- oder -O-CO-) können auch durch Veresterung entsprechender Carbonsäuren (oder ihrer reaktionsfähigen Derivate) mit Alkoholen bzw. Phenolen (oder ihren reaktionsfähigen Derivaten) erhalten werden. Die Veresterung von Säuren mit Alkoholen bzw. Phenolen kann auch mit DCC/DMAP durchgeführt werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate in Betracht. Darin ist das Metall vorzugsweise ein Alkalimetall wie Na oder K.

Dioxanderivate bzw. Dithianderivate der Formel I (worin eine der Gruppen $A^1$ und/oder $A^2$ eine 1,3-Dioxan-2,5-diyl-Gruppe bzw. 1,3-Dithian-2,5-diyl-Gruppe bedeutet) werden zweckmäßig durch Reaktion eines entsprechenden Aldehyds (oder eines seiner reaktionsfähigen Derivate) mit einem entsprechenden 1,3-Diol (oder einem seiner reaktionsfähigen Derivate) bzw. einem entsprechenden 1,3-Dithiol hergestellt, vorzugsweise in Gegenwart eines inerten Lösungsmittels wie Benzol oder Toluol und/oder eines Katalysators, z.B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester und die Dithiole durch Umsetzung entsprechender Dihalogenide mit NaSH erhältlich.

Zur Herstellung von Nitrilen der Formel I (worin $R^1$ CN bedeutet und/oder worin $A^3$ und/oder $A^4$ und/oder $A^1$ und/oder $A^2$ durch mindestens eine CN-Gruppe substituiert ist) können entsprechende Säureamide dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Ether der Formel I (worin $R^1$ eine Alkylgruppe bedeutet, worin eine oder zwei $CH_2$-Gruppen durch O-Atome ersetzt sind, und/oder worin $Z^2$ und/oder $Z^1$ eine $-OCH_2$- oder eine $-CH_2O$-Gruppe ist) sind durch Veretherung entsprechender Phenole, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Zur Herstellung von lateral durch Halogen substituierte Verbindungen der Formel I können im Prinzip alle Methoden, die für die Herstellung solcher Verbindungen bekannt sind, angewendet werden. Der Fachmann kann die erforderlichen Synthesevarianten nach Routinemethoden ableiten.

Die erfindungsgemäßen flüssigkristallinen Phasen bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Biscyclohexylbenzole, 4,4'-Bis-cyclohexylbiphenyle, Phenyl- oder Cyclophexylpyrimidine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, gegebenenfalls halogenierten Stilbene, Benzylphenylether und substituierten Zimtsäuren.

Die wichtigsten als Bestandteile derartiger flüssigkristalliner Phasen in Frage kommenden Verbindungen lassen sich durch die Formel IV charakterisieren,

$$R^6\text{-L-G-E-}R^7 \quad \text{IV}$$

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | -CH=CH- | -N(O)=N- |
| | -CH=CY- | -CH=N(O)- |
| | -C≡C- | $-CH_2-CH_2-$ |
| | -CO-O- | $-CH_2-O-$ |
| | -CO-S- | $-CH_2-S-$ |
| | -CH=N- | -COO-Phe-COO- |

oder eine C-C-Einfachbindung,

Y Halogen, vorzugsweise Chlor, oder -CN, und

$R^6$ und $R^7$ Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind $R^6$ und $R^7$ voneinander verschieden, wobei einer dieser Reste meist eine Alkyl- oder Alkoxygruppe ist. Auch auch andere Varianten der vorgesehenen Substituenten sind gebräuchlich. Viele solcher Substanzen oder auch Gemische davon sind im Handel erhältlich. Alle diese Substanzen sind nach literaturbekannten Methoden erhältlich.

Die erfindungsgemäßen Phasen enthalten etwa 0,1 bis 99 vorzugsweise 10 bis 95 %, einer oder mehrer Verbindungen der Formel I. Weiterhin bevorzugt sind erfindungsgemäße flüssigkristalline Phasen, enthaltend 0,1-40, vorzugsweise 0,5-30 % einer oder mehrerer Verbindungen der Formel I.

Die Verbindungen der Formel I können auch als Komponenten smektischer oder chiral getilteter smektischer flüssigkristalliner Phasen verwendet werden. Diese Phasen sind bevorzugt chiral getiltete smektische flüssigkristalline Phasen, deren achirale Basismischung neben Verbindungen der Formel I mindestens eine andere Komponente mit negativer oder betragsmäßig kleiner positiver dielektrischen Anisotropie enthält. Diese weitere(n) Komponente(n) der achiralen Basismischung kann (können) zu 1 bis 50 %, vorzugsweise 10 bis 25 %, der Basismischung ausmachen.

Die Herstellung der erfindungsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vg. z.B. I. Haller et al., Mol. Cryst. Liq. Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Beispiel 1

0,2 m 4-Trifluormethoxybenzaldehyd und 0,2 m 2-Methyl-5-methylpyridin werden zusammen mit 3 g Zinkchlorid 2 Tage auf 200° erhitzt. Der Verlauf der Reaktion kann mit Hilfe der Dünnschichtchromatographie verfolgt werden. Nach beendeter Reaktion wird überschüssiges Ausgangsmaterial abdestilliert und der Rückstand durch Kristallisation oder Chromatographie gereinigt.

0,1 m des so erhaltenen Stilbenderivats werden in 200 ml Eisessig bei Raumtemperatur unter Rühren mit 0,1 m $Br_2$ bromiert. Nach beendeter Zugabe erhitzt man kurz zum Sieden. Danach wird der Eisessig abgedampft und der Rückstand mit 200 ml tert-Butanol versetzt. Zu diesem Gemisch gibt man bei Raumtemperatur 0,4 m Kalium-tert-butanolat und erhitzt danach 3 Stunden zum Sieden. Nach Abkühlen wird Wasser zugesetzt und mit Ether extrahiert. Nach Aufarbeitung der organischen Phase und Reinigung durch Chromatographie erhält man 1-(4-Trifluormethoxyphenyl)-2-(5-methyl-pyridin-2-yl)-acetylen.

Analog werden hergestellt:

1-(4-Trifluormethoxyphenyl)-2-(5-ethyl-pyridin-2-yl)-acetylen
1-(4-Trifluormethoxyphenyl)-2-(5-propyl-pyridin-2-yl)-acetylen
1-(4-Trifluormethoxyphenyl)-2-(5-butyl-pyridin-2-yl)-acetylen
1-(4-Trifluormethoxyphenyl)-2-(5-pentyl-pyridin-2-yl)-acetylen
1-(4-Trifluormethoxyphenyl)-2-(5-hexyl-pyridin-2-yl)-acetylen
1-(4-Trifluormethoxyphenyl)-2-(5-heptyl-pyridin-2-yl)-acetylen
1-(4-Trifluormethoxyphenyl)-2-(5-octyl-pyridin-2-yl)-acetylen
1-(4-Trifluormethoxyphenyl)-2-(5-methoxy-pyridin-2-yl)-acetylen
1-(4-Trifluormethoxyphenyl)-2-(5-ethoxy-pyridin-2-yl)-acetylen
1-(4-Trifluormethoxyphenyl)-2-(5-propoxy-pyridin-2-yl)-acetylen
1-(4-Trifluormethoxyphenyl)-2-(5-butoxy-pyridin-2-yl)-acetylen
1-(4-Trifluormethoxyphenyl)-2-(5-pentyloxy-pyridin-2-yl)-acetylen
1-(4-Trifluormethoxyphenyl)-2-(5-hexyloxy-pyridin-2-yl)-acetylen
1-(4-Trifluormethoxyphenyl)-2-(5-heptyloxy-pyridin-2-yl)-acetylen
1-(4-Trifluormethoxyphenyl)-2-(5-octyloxy-pyridin-2-yl)-acetylen

Beispiel 2

0,2 m trans-4-(trans-4-Propylcyclohexyl)-cyclohexylmethyl-triphenylphosphoniumiodid und 0,2 m 4-Trifluormethoxybenzaldehyd werden zusammen in 250 ml THF nach Wittig bei 0-5° mit einer Lösung von 0,2 m Kalium-tert.butanolat in 150 ml THF versetzt. Nach 1 Stunde Rühren bei Raumtemperatur neutralisiert man mit verd. HCl, filtriert das Phosphoniumoxid ab und arbeitet das Filtrat extraktiv auf. Nach Reinigung durch Kristallisation erhält man das entsprechende Stilbenprodukt.

0,1 m dieser Stilbenverbindung werden in 100 ml Acetonitril bei 0-5° mit 0,1 m $Br_2$ bromiert. Das ausgefallene Produkt wird abfiltriert und getrocknet. Dann wird das Dibromid mit 0,2 m Kalium-tert.butanolat und 150 ml tert. Butanol 3 Stunden am Rückfluß erhitzt. Nach dem Abkühlen gießt man auf Wasser und nimmt das Produkt in Petrolether auf.

Nach extraktiver Aufarbeitung und Reinigung durch Kristallisation erhält man 1-[trans-4-(trans-4-Propylcyclohexyl)cyclohexyl]-2-(4-trifluormethoxyphenyl)-acetylen.

Analog werden hergestellt:
1-[trans-4-(trans-4-Methylcyclohexyl)cyclohexyl]-2-(4-trifluormethoxyphenyl)-acetylen
1-[trans-4-(trans-4-Ethylcyclohexyl)cyclohexyl]-2-(4-trifluormethoxyphenyl)-acetylen
1-[trans-4-(trans-4-Butylcyclohexyl)cyclohexyl]-2-(4-trifluormethoxyphenyl)-acetylen
1-[trans-4-(trans-4-Pentylcyclohexyl)cyclohexyl]-2-(4-trifluormethoxyphenyl)-acetylen
1-[trans-4-(trans-4-Hexylcyclohexyl)cyclohexyl]-2-(4-trifluormethoxyphenyl)-acetylen
1-[trans-4-(trans-4-Heptylcyclohexyl)cyclohexyl]-2-(4-trifluormethoxyphenyl)-acetylen
1-[trans-4-(trans-4-Octylcyclohexyl)cyclohexyl]-2-(4-trifluormethoxyphenyl)-acetylen
1-(trans-4-Propylcyclohexyl)-2-(4-trifluormethoxyphenyl)-acetylen
1-(trans-4-Ethylcyclohexyl)-2-(4-trifluormethoxyphenyl)-acetylen
1-(trans-4-Butylcyclohexyl)-2-(4-trifluormethoxyphenyl)-acetylen
1-(trans-4-Pentylcyclohexyl)-2-(4-trifluormethoxyphenyl)-acetylen
1-(trans-4-Hexylcyclohexyl)-2-(4-trifluormethoxyphenyl)-acetylen
1-(trans-4-Heptylcyclohexyl)-2-(4-trifluormethoxyphenyl)-acetylen
1-(trans-4-Octylcyclohexyl)-2-(4-trifluormethoxyphenyl)-acetylen
1-[trans-4-(4-Propylphenyl)cyclohexyl]-2-(4-trifluormethoxyphenyl)-acetylen
1-[trans-4-(4-Ethylphenyl)cyclohexyl]-2-(4-trifluormethoxyphenyl)-acetylen
1-[trans-4-(4-Butylphenyl)cyclohexyl]-2-(4-trifluormethoxyphenyl)-acetylen
1-[trans-4-(4-Pentylphenyl)cyclohexyl]-2-(4-trifluormethoxyphenyl)-acetylen
1-[trans-4-(4-Hexylphenyl)cyclohexyl]-2-(4-trifluormethoxyphenyl)-acetylen
1-[trans-4-(4-Heptylphenyl)cyclohexyl]-2-(4-trifluormethoxyphenyl)-acetylen
1-[trans-4-(4-Octylphenyl)cyclohexyl]-2-(4-trifluormethoxyphenyl)-acetylen

Beispiel 3

Analog Beispiel 2 erhält man aus 4-(1,1,2,2-Tetrafluorethoxy)benzaldehyd und trans-4-Propylcyclohexylmethylphosphoniumiodid das entsprechende 1-(trans-4-Propylcyclohexyl)-2-[4-(1,1,2,2-tetrafluorethoxy)phenyl]-acetylen.

Analog werden hergestellt:

1-(trans-4-Ethylcyclohexyl)-2-[4-(1,1,2,2-tetrafluorethoxy)phenyl]-acetylen
1-(trans-4-Methylcyclohexyl)-2-[4-(1,1,2,2-tetrafluorethoxy)phenyl]-acetylen
1-(trans-4-Butylcyclohexyl)-2-[4-(1,1,2,2-tetrafluorethoxy)phenyl]-acetylen
1-(trans-4-Pentylcyclohexyl)-2-[4-(1,1,2,2-tetrafluorethoxy)phenyl]-acetylen
1-(trans-4-Hexylcyclohexyl)-2-[4-(1,1,2,2-tetrafluorethoxy)phenyl]-acetylen
1-(trans-4-Heptylcyclohexyl)-2-[4-(1,1,2,2-tetrafluorethoxy)phenyl]-acetylen
1-(trans-4-Octylcyclohexyl)-2-[4-(1,1,2,2-tetrafluorethoxy)phenyl]-acetylen
1-[trans-4-(trans-4-Propylcyclohexyl)cyclohexyl]-2-[4-(1,1,2,2-tetrafluorethoxy)phenyl]-acetylen
1-[trans-4-(trans-4-Methylcyclohexyl)cyclohexyl]-2-[4-(1,1,2,2-tetrafluorethoxy)phenyl]-acetylen
1-[trans-4-(trans-4-Butylcyclohexyl)cyclohexyl]-2-[4-(1,1,2,2-tetrafluorethoxy)phenyl]-acetylen
1-[trans-4-(trans-4-Pentylcyclohexyl)cyclohexyl]-2-[4-(1,1,2,2-tetrafluorethoxy)phenyl]-acetylen
1-[trans-4-(trans-4-Hexylcyclohexyl)cyclohexyl]-2-[4-(1,1,2,2-tetrafluorethoxy)phenyl]-acetylen
1-(trans-4-(trans-4-Heptylcyclohexyl)cyclohexyl]-2-(4-(1,1,2,2-tetrafluorethoxy)phenyl]-acetylen
1-(trans-4-(trans-4-Octylcyclohexyl)cyclohexyl]-2-(4-(1,1,2,2-tetrafluorethoxy)phenyl]-acetylen
1-[trans-4-(4-Propylphenyl)cyclohexyl]-2-[4-(1,1,2,2-tetrafluorethoxy)phenyl]-acetylen
1-[trans-4-(4-Methylphenyl)cyclohexyl]-2-[4-(1,1,2,2-tetrafluorethoxy)phenyl]-acetylen
1-[trans-4-(4-Butylphenyl)cyclohexyl]-2-[4-(1,1,2,2-tetrafluorethoxy)phenyl]-acetylen
1-[trans-4-(4-Pentylphenyl)cyclohexyl]-2-[4-(1,1,2,2-tetrafluorethoxy)phenyl]-acetylen
1-[trans-4-(4-Hexylphenyl)cyclohexyl]-2-[4-(1,1,2,2-tetrafluorethoxy)phenyl]-acetylen
1-[trans-4-(4-Heptylphenyl)cyclohexyl]-2-[4-(1,1,2,2-tetrafluorethoxy)phenyl]-acetylen
1-[trans-4-(4-Octylphenyl)cyclohexyl]-2-(4-(1,1,2,2-tetrafluorethoxy)phenyl]-acetylen.

Beispiel 4

Analog Beispiel 1 erhält man aus 4-(1,1,2,2-Tetrafluorethoxy)benzaldehyd und 2-Methyl-5-methylpyridin das entsprechende 1-[4-(1,1,2,2-Tetrafluorethoxy)phenyl]-2-(5-methyl-pyridin-2-yl)-acetylen.

Analog werden hergestellt:

1-[4-(1,1,2,2-Tetrafluorethoxy)phenyl]-2-(5-ethyl-pyridin-2-yl)-acetylen
1-[4-(1,1,2,2-Tetrafluorethoxy)phenyl]-2-(5-propyl-pyridin-2-yl)-acetylen
1-[4-(1,1,2,2-Tetrafluorethoxy)phenyl]-2-(5-butyl-pyridin-2-yl)-acetylen
1-[4-(1,1,2,2-Tetrafluorethoxy)phenyl]-2-(5-pentyl-pyridin-2-yl)-acetylen
1-[4-(1,1,2,2-Tetrafluorethoxy)phenyl]-2-(5-hexyl-pyridin-2-yl)-acetylen
1-[4-(1,1,2,2-Tetrafluorethoxy)phenyl]-2-(5-heptyl-pyridin-2-yl)-acetylen
1-[4-(1,1,2,2-Tetrafluorethoxy)phenyl]-2-(5-octyl-pyridin-2-yl)-acetylen
1-[4-(1,1,2,2-Tetrafluorethoxy)phenyl]-2-(5-methoxy-pyridin-2-yl)-acetylen
1-[4-(1,1,2,2-Tetrafluorethoxy)phenyl]-2-(5-ethoxy-pyridin-2-yl)-acetylen
1-[4-(1,1,2,2-Tetrafluorethoxy)phenyl]-2-(5-propoxy-pyridin-2-yl)-acetylen
1-[4-(1,1,2,2-Tetrafluorethoxy)phenyl]-2-(5-butoxy-pyridin-2-yl)-acetylen
1-[4-(1,1,2,2-Tetrafluorethoxy)phenyl]-2-(5-pentyloxy-pyridin-2-yl)-acetylen
1-[4-(1,1,2,2-Tetrafluorethoxy)phenyl]-2-(5-hexyloxy-pyridin-2-yl)-acetylen
1-[4-(1,1,2,2-Tetrafluorethoxy)phenyl]-2-(5-heptyloxy-pyridin-2-yl)-acetylen
1-[4-(1,1,2,2-Tetrafluorethoxy)phenyl]-2-(5-octyloxy-pyridin-2-yl)-acetylen.

Beispiel 5

Ausgehend von 4-Trifluormethoxybenzaldehyd erhält man durch Umsetzung (analog Organic Syntheses, S. 327) mit Malonsäure die entsprechende Zimtsäure. Diese Verbindung wird durch Umsetzung mit 1. $SOCl_2$, 2. $NH_3$ und 3. $POCl_3$ in das Nitril überführt.

Analog der von P.E. Fanta et al. in J. Am. Chem. Soc., Vol. 78, 1434 beschriebenen Methode wird das Nitril in ein Amidin überführt, das mit einem Natriumsalz des 2-Pentylmalonsäurealdehyds in das 1-(4-Trifluormethoxyphenyl)-2-(5-pentyl-pyrimidin-2-yl)-ethen überführt wird.

Dieses Stilbenderivat wird in Acetonitril bei 0-5° bromiert und anschließend mit Kalium-tert.butanolat in tert. Butanol dehydrobromiert. Nach üblicher Aufarbeitung und Reinigung erhält man 1-(4-Trifluormethoxyphenyl)-2-(5-pentyl-pyrimidin-2-yl)-ethin.

Analog werden hergestellt:

1-(4-Trifluormethoxyphenyl)-2-(5-ethyl-pyrimidin-2-yl)-ethin
1-(4-Trifluormethoxyphenyl)-2-(5-methyl-pyrimidin-2-yl)-ethin
1-(4-Trifluormethoxyphenyl)-2-(5-propyl-pyrimidin-2-yl)-ethin

1-(4-Trifluormethoxyphenyl)-2-(5-butyl-pyrimidin-2-yl)-ethin
1-(4-Trifluormethoxyphenyl)-2-(5-pentyl-pyrimidin-2-yl)-ethin
1-(4-Trifluormethoxyphenyl)-2-(5-hexyl-pyrimidin-2-yl)-ethin
1-(4-Trifluormethoxyphenyl)-2-(5-heptyl-pyrimidin-2-yl)-ethin
1-(4-Trifluormethoxyphenyl)-2-(5-octyl-pyrimidin-2-yl)-ethin
1-[4-(1,1,2,2-Tetrafluorethoxy)phenyl)-2-(5-pentyl-pyrimidin-2-yl)-ethin
1-[4-(1,1,2,2-Tetrafluorethoxy)phenyl)-2-(5-ethyl-pyrimidin-2-yl)-ethin
1-[4-(1,1,2,2-Tetrafluorethoxy)phenyl)-2-(5-methyl-pyrimidin-2-yl)-ethin
1-[4-(1,1,2,2-Tetrafluorethoxy)phenyl-1)-2-(5-propyl-pyrimidin-2-yl)-ethin
1-[4-(1,1,2,2-Tetrafluorethoxy)phenyl)-2-(5-butyl-pyrimidin-2-yl)-ethin
1-[4-(1,1,2,2-Tetrafluorethoxy)phenyl)-2-(5-hexyl-pyrimidin-2-yl)-ethin
1-[4-(1,1,2,2-Tetrafluorethoxy)phenyl)-2-(5-heptyl-pyrimidin-2-yl)-ethin
1-[4-(1,1,2,2-Tetrafluorethoxy)phenyl)-2-(5-octyl-pyrimidin-2-yl)-ethin

Beispiel A Man stellt eine flüssigkristalline Phase her aus

9 % r-1-Cyan-cis-4-(trans-4-propylcyclohexyl)-1-propyl-cyclohexan,
5 % r-1-Cyan-1-propyl-cis-4-(4′-propylbiphenyl-4-yl)-cyclohexan,
26 % 2-Fluor-4-ethyl-4′-[2-(trans-4-propyl-cyclohexyl)ethyl]-biphenyl,
25 % 2-Fluor-4-pentyl-4′-[2-(trans-4-propyl-cyclohexyl)ethyl]-biphenyl,
23 % 2-Fluor-4-ethyl-4′-[2-(trans-4-pentyl-cyclohexyl)ethyl]-biphenyl,
5 % 4-(trans-4-Propylcyclohexyl)-2′-fluor-4′-(trans-4-propylcyclohexyl)-biphenyl
und
7 % 1-(4-Trifluormethoxyphenyl)-2-(trans-4-ethylcyclohexyl)-acetylen.

**Patentansprüche**

1. Ethirderivate der Formel I

$$R^1\text{-}(A^1\text{-}Z^1)_m\text{-}A^3\text{-}C{\equiv}C\text{-}A^4\text{-}(Z^2\text{-}A^2)_n\text{-}R^2 \quad \text{I}$$

worin

$R^1$ einen unsubstituierten, einen einfach durch -CN oder einen mindestens einfach durch Halogen substituierten Alkyl- oder Alkenylrest mit 1 bis 15 C-Atomen, wobei in diesen Resten auch eine oder mehrere $CH_2$-Gruppen jeweils unabhängig voneinander durch -O-, -S-, -CO-, -O-CO-, -O-COO-, -CO-O- oder -C≡C- so ersetzt sein können, daß Heteroatome nicht direkt miteinander verknüpft sind, H, Halogen, -CN oder -NCS,

$R^2$ $-OCF_3$, $-OC_2F_5$ oder $-OC_2F_4H$,

$A^1$ und $A^2$ jeweils unabhängig voneinander einen

a) 1,4-Phenylenrest, worin auch eine oder mehrere CH-Gruppen durch N ersetzt sein können,

b) trans-1,4-Cyclohexylenrest, worin auch eine oder zweinicht benachbarte $CH_2$-Gruppen durch -O- und/oder -S- ersetzt sein können,

c) Rest aus der Gruppe 1,4-Cyclohexenylen, 1,4-Cyclohexadienylen oder 1,4-Bicyclo-(2.2.2)-octylen,

wobei die Reste a) und b) ein- oder mehrfach durch Halogen, Cyano und/oder $CH_3$ substituiert sein können,

$Z^1$ und $Z^2$ jeweils unabhängig voneinander -CO-O-, -O-CO-, $-CH_2O-$, $-OCH_2-$, $-CH_2CH_2-$, -C≡C- oder eine Einfachbindung,

m und n jeweils unabhängig voneinander 0 oder 1,

und

$A^3$ und $A^4$ jeweils unabhängig voneinander einen

a) 1,4-Phenylen- oder 4,4′-Biphenylenrest, worin auch eine oder mehrere CH-Gruppen durch N ersetzt sein können,

b) trans-1,4-Cyclohexylenrest,

c) Rest aus der Gruppe 1,4-Cyclohexenylen, 1,4-Cyclohexadienylen oder 1,4-Bicyclo-(2.2.2)octylen, wobei die Reste a) und b) ein- oder mehrfach durch Halogen, Cyano und/oder $CH_3$ substituiert sein können, bedeutet, mit der Maßgabe daß, in mindestens einer der Gruppen $A^3$ oder $A^4$ mindestens eine CH-Gruppe durch N ersetzt ist und/oder mindestens eine der Gruppen $A^3$ und $A^4$ trans-1,4-Cyclohexylen oder 1,4-Bicyclo(2.2.2)octylen ist und/oder mindestens eine der Gruppen $A^1$, $A^2$, $A^3$ und $A^4$ 1,4-Cyclohexenylen oder 1,4-Cyclohexadienylen ist.

2. Verwendung der Verbindungen der Formel I nach Anspruch 1 als Komponenten flüssigkristalliner Phasen.

3. Flüssigkristalline Phase mit mindestens zwei flüssigkristallinen Komponenten, dadurch gekennzeichnet, daß sie mindestens eine Verbindung der Formel I nach Anspruch 1 enthält.

4. Flüssigkristallanzeigeelement, dadurch gekennzeichnet, daß es eine flüssigkristalline Phase nach Anspruch 3 enthält.

## Claims

1. Ethyne derivatives of the formula I

$$R^1-(A^1-Z^1-)_m-A^3-C{\equiv}C-A^4-(Z^2-A^2)_n-R^2 \quad I$$

wherein

$R^1$ is an alkyl or alkenyl radical having 1 to 15 C atoms which is unsubstituted, monosubstituted by -CN or at least monosubstituted by halogen, it also being possible for one or more $CH_2$ goups in these radicals in each case independently of one another to be replaced by -O-, -S-, -CO-, -O-CO-, -O-COO-, -CO-O- or -C≡C- such that heteroatoms are not linked directly to one another, H, halogen, -CN or -NCS,

$R^2$ is $-OCF_3$, $-OC_2F_5$ or $-OC_2F_4H$,

$A^1$ and $A^2$ in each case independently of one another are a

a) 1,4-phenylene radical, wherein one or more CH groups can also be replaced by N,

b) trans-1,4-cyclohexylene radical, wherein one or two non-adjacent CH2 groups can also be replaced by -O- and/or -S-,

c) radical from the group comprising 1,4-cyclohexenylene, 1,4-cyclohexadienylene or 1,4-bicyclo-(2.2.2)-octylene,

it being possible for the radicals a) and b) to be substituted once or more than once by halogen, cyano and/or $CH_3$,

$Z^1$ and $Z^2$ in each case independently of one another are -CO-O-, -O-CO-, $-CH_2O-$, $-OCH_2-$, $-CH_2CH_2-$, -C≡C- or a single bond,

m and n in each case independently of one another are 0 or 1,

and

$A^3$ and $A^4$ in each case independently of one another are a

a) 1,4-phenylene or 4,4'-biphenylene radical, wherein one or more CH groups can also be replaced by N,

b) trans-1,4-cyclohexylene radical,

c) radical from the group comprising 1,4-cyclohexenylene, 1,4-cyclohexadienylene or 1,4-bicyclo(2.2.2)octylene,

it being possible for the radicals a) and b) to be substituted once or more than once by halogen, cyano and/or $CH_3$,

with the proviso that in at least one of the groups $A^3$ or $A^4$ at least one CH group is replaced by N and/or at least one of the groups $A^3$ and $A^4$ is trans-1,4-cyclohexylene or 1,4-bicyclo(2.2.2)octylene and/or at least one of the groups $A^1$, $A^2$, $A^3$ and $A^4$ is 1,4-cyclohexenylene or 1,4-cyclohexadienylene.

2. Use of the compounds of the formula I according to Claim 1 as components of liquid crystal phases.

3. Liquid crystal phase with at least two liquid crystal components, characterized in that it contains at least one compound of the formula I according to Claim 1.

4. Liquid crystal display element, characterized in that it contains a liquid crystal phase according to Claim 3.

## Revendications

1. Dérivés d'éthine de formule I

$$R^1-(A^1-Z^1-)_m-A^3-C{\equiv}C-A^4-(Z^2-A^2)_n-R^2 \quad I$$

où

$R^1$ représente un reste alkyle ou alkényle, avec de 1 à 15 atomes de C, non substitué, substitué une fois avec -CN ou substitué au moins une fois par un halogène, de telle sorte que dans ces restes aussi un ou plusieurs groupes $Ch_2$, à chaque fois de façon indépendante les uns des autres, peuvent être substitués par -O-, -S-, -CO-, -O-CO-, -O-COO-, -CO-O- ou -C≡C- de telle sorte que les hétéroatomes ne soient par reliés directement entr'eux, un H, un halogène, -CN ou -NCS,

$R^2$ représente $-OCF_3$, $-OC_2F_5$ ou $-OC_2F_4H$,

$A^1$ et $A^2$, chaque fois indépendamment l'un de l'autre, représentent chacun

a) un reste 1,4-phényle, où aussi un ou plusieurs groupes CH peuvent être remplacés par N,

b) un reste trans-1,4-cyclohexyle, où aussi un ou deux groupes $CH_2$ non voisins peuvent être remplacés par -O- et/ou -S-,

c) un reste du groupe 1,4-cyclohexénylène, 1,4-cyclohexadiénylène ou 1,4-bicyclo-(2.2.2)-octylène,

où les restes a) et b) peuvent être substitués une ou plusieurs fois par un halogène, un cyano et/ou $CH_3$,

$Z^1$ et $Z^2$, indépendamment l'un de l'autre, représentent chacun -CO-O-, -O-CO-, $-CH_2O-$, $-OCH_2-$, $-CH_2-CH_2-$, -C≡C- ou une liaison simple,

m et n, indépendamment l'un de l'autre, représentent chacun 0 ou 1,

et

$A^3$ et $A^4$, indépendamment l'un de l'autre, représentent chacun

a) un reste 1,4-phénylène ou un reste 4,4'-biphénylène, où aussi un ou plusieurs groupes CH peuvent être remplacés par N,

b) un reste trans-1,4-cyclohexylène,

c) un reste du groupe 1,4-cyclohexénylène, 1,4-cyclohexadiénylène ou 1,4-bicyclo(2.2.2)-octylène, où les restes a) et b) peuvent être substitués une ou plusieurs fois par un halogène, un cyano et/ou $CH_3$,

étant entendu que, dans au moins un des groupes $A^3$ ou $A^4$, au moins un groupe CH est remplacé par N, et/ou au moins un des groupes $A^3$ et $A^4$ est un trans-1,4-cyclohexylène ou 1,4-bicyclo(2.2.2)octylène, et/ou au moins un des groupes $A^1$, $A^2$, $A^3$ et $A^4$ est un 1,4-cyclohexénylène ou un 1,4-cyclohexadiénylène.

2. Utilisation des composés de formule I selon la revendication 1 comme composants de phases à cristaux liquides.

3. Phase à cristaux liquides avec au moins deux composants à cristaux liquides, caractérisée en ce qu'elle comporte au moins un composé de formule I selon la revendication 1.

4. Elément d'affichage à cristaux liquides, caractérisé en ce qu'il comporte une phase à cristaux liquides selon la revendication 3.